# EUROPEAN PATENT SPECIFICATION

(11) **EP 3 351 542 B1**
(45) Date of publication and mention of the grant of the patent: **17.11.2021**
(21) Application number: 16846662.1
(22) Date of filing: 16.09.2016
(51) Int. Cl.: C07D 401/12, A61K 31/5377, A61P 29/00, A61P 37/08, A61P 43/00

(54) **NEW CRYSTAL OF PIPERAZINE COMPOUND**
NEUES KRISTALL EINER PIPERAZINVERBINDUNG
NOUVEAU CRISTAL DE COMPOSÉ DE PIPÉRAZINE

(30) Priority: 17.09.2015 JP 2015184022
(43) Date of publication of application: 25.07.2018
(73) Proprietor: Taiho Pharmaceutical Co., Ltd., Chiyoda-ku Tokyo 101-8444 (JP)
(72) Inventor: AOKI, Shinichi, Kodama-gun Saitama 367-0241 (JP)
(74) Representative: Hoffmann Eitle
(86) International application number: PCT/JP2016/077547
(87) International publication number: WO 2017/047791

(56) References cited:
- EP-A1- 2 407 466
- EP-A1- 2 898 886
- WO-A1-2010/104024
- WO-A1-2014/046129
- CAIRA M R ED - MONTCHAMP JEAN-LUC: "CRYSTALLINE POLYMORPHISM OF ORGANIC COMPOUNDS", TOPICS IN CURRENT CHEMISTRY; [TOPICS IN CURRENT CHEMISTRY], SPRINGER, BERLIN, DE, vol. 198, 1 January 1998 (1998-01-01), pages 163-208, XP001156954, ISSN: 0340-1022, DOI: 10.1007/3-540-69178-2_5
- Sadao Nishigaki: "Passage; CHOZAIGAKU. KISO TO OYO", Chozaigaku -Kiso to Oyo, 20 September 1977 (1977-09-20), pages 142-145, XP009510252, Japam
- Yuki Nakai, Manabu Hanano: "Passages, SHIN SEIZAIGAKU [New pharmaceutical science]", SHIN SEIZAIGAKU [New pharmaceutical science], 25 April 1984 (1984-04-25), pages 102-103 , 232 to 233, XP009510026, ISBN: 4-525-77291-3
- Shin·Yakuzaigaku Soron, 10 April 1987 (1987-04-10), page 111, XP002966372,
- HIROSHI OSHIMA: "Kessho Takei·Gitakei no Sekishutsu Kyodo to Seigyo", PHARM STAGE, vol. 6, no. 10, 15 January 2007 (2007-01-15), pages 48-53, XP008184800,
- NORIYUKI TAKATA: "Soyaku Dankai ni Okeru Gen'yaku Form Screening to Sentaku", PHARM STAGE, vol. 6, no. 10, 15 January 2007 (2007-01-15), pages 20-25, XP008145548,
- MITSUHISA YAMANO: "Approach to Crystal Polymorph in Process Research of New Drug", Journal of Synthetic Organic Chemistry, vol. 65, no. 9, 1 September 2007 (2007-09-01), XP055358201, Japan
- Kaoru Kaoru: "Recrystallization method and precipitation method", JIKKEN KAGAKU KOZA (ZOKU) 2 BUNRI TO SEISEI [Experimental Chemistry Course (continued) 2 Separation and purification], 25 January 1967 (1967-01-25), pages 159-178 , 186 to 187, XP009509740,

## Description

### Technical Field

The present invention relates to a novel crystal of a piperazine compound. More specifically, the present invention relates to a novel crystal of a piperazine compound that can be stably provided as a crystal of an active pharmaceutical ingredient for use in the manufacture of medicines, while ensuring sufficient reproducibility, and that has excellent storage stability; and the invention relates to a useful pharmaceutical composition containing the crystal as an active ingredient.

### Background Art

In general, when a compound is used as an active ingredient of a pharmaceutical product, it is necessary to stably obtain a single crystal having a constant quality while ensuring sufficient reproducibility. Further, it is preferred that the resulting single crystal has excellent stability.

Patent Document 1 discloses, as a compound having a prostaglandin D synthase inhibiting action, 4-((1-methylpyrrol-2-yl)-carbonyl)-N-(4-(4-morpholin-1-yl-carbonylpiperidin-1-yl)-phenyl)-1-piperazinecarboxamide (which hereinafter may also be referred to as "Compound (1)") represented by Formula (1) below.

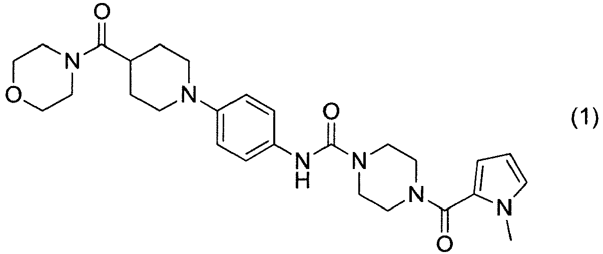

Patent Document 1 discloses that Compound (1) exhibits a hematopoietic prostaglandin D synthase (H-PGDS) inhibiting effect in humans. Compound (1) is currently clinically developed.

The method for producing Compound (1) disclosed in Patent Document 1 comprises purification of an obtained reaction product using silica-gel column chromatography. The obtained compound was in the form of a foamy substance (amorphous).

Further, Patent Document 2 discloses a formulation example of a monohydrate of Compound (1).

As described above, preferable crystal forms of Compound (1) as a crystal of an active pharmaceutical ingredient for use in the manufacture of pharmaceutical products have been completely unknown.

### Citation List

### Patent Documents

Patent Document 1: WO2010/104024 (corresponding to EP 2 407 466 A1)
Patent Document 2: WO2014/046129 (corresponding to EP 2 898 886 A1)

### Summary of Invention

### Technical Problem

An object of the present invention is to provide a crystal of Compound (1) having a prostaglandin D synthase inhibiting action that has excellent storage stability as an active pharmaceutical ingredient for use in the manufacture of pharmaceutical products, and preferably that can be obtained with sufficient reproducibility.

### Solution to Problem

The inventors of the present invention conducted extensive research to attain the above object, and succeeded in obtaining novel crystal I by performing recrystallization at a temperature equal to or higher than room temperature by using an organic solvent that has been generally used for a crude product of Compound (1), such as an alcohol or ketone-based solvent. The inventors also succeeded in obtaining novel crystal II by performing suspension washing of Compound (1) at a temperature of 100°C or more by using a generally used organic solvent. More specifically, the inventors found novel crystals I and II having excellent absorbability and stability, and found, in particular, that crystal I has excellent storage stability and can be obtained while ensuring sufficient reproducibility. With this finding, the inventors completed the present invention.

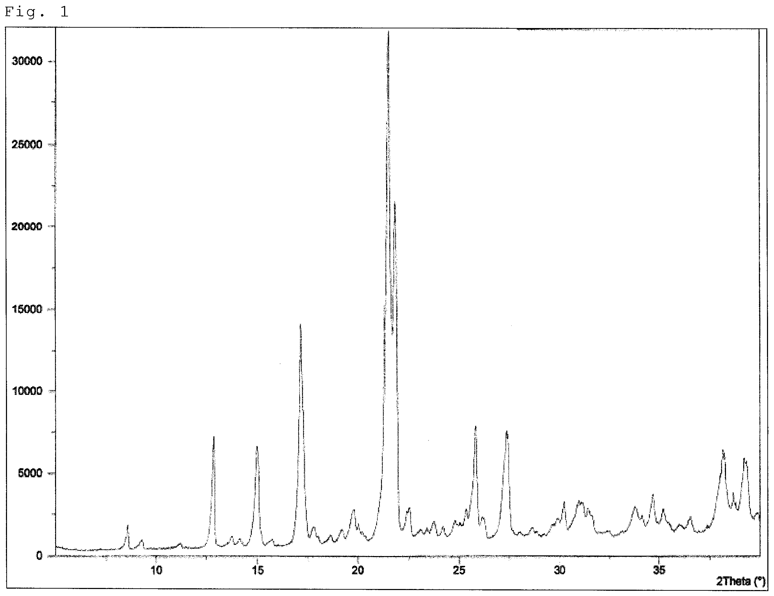

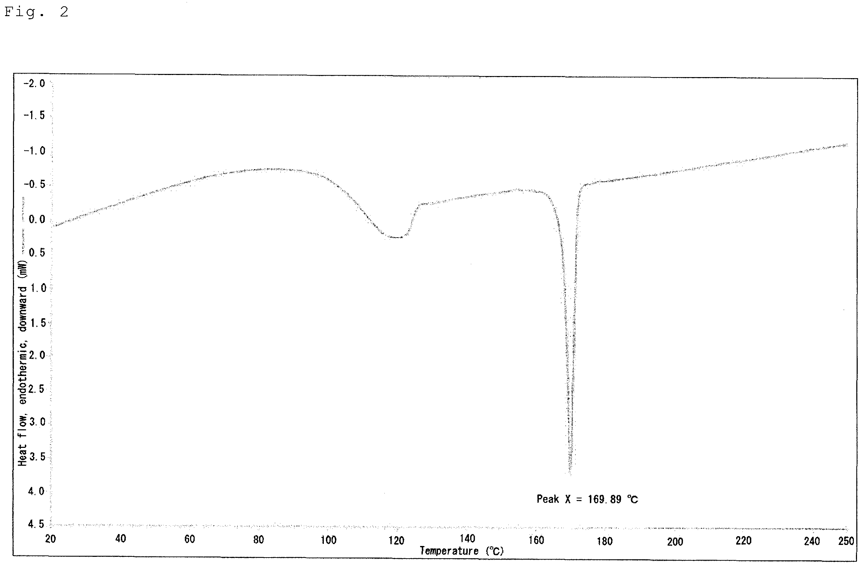

Specifically, the present invention provides Items 1 to 5 below.
1. A crystal of monohydrate of 4-((1-methylpyrrol-2-yl)-carbonyl)-N-(4-(4-morpholin-1-yl-carbonylpiperidin-1-yl)-phenyl)-1-piperazinecarboxamide (Compound (1)) having at least 2 peaks of diffraction angle (2θ±0.2°) selected from 12.8°, 15.0°, 17.2°, 19.8°, 21.5°, 21.8°, 22.5°, 25.8°, and 27.5° in a powder X-ray diffraction spectrum, wherein the chemical purity of Compound (1) in the crystal is 99% or more, as measured by HPLC.
2. The crystal according to Item 1, wherein the crystal has at least 5 peaks of diffraction angle (2θ±0.2°) selected from 12.8°, 15.0°, 17.2°, 19.8°, 21.5°, 21.8°, 22.5°, 25.8°, and 27.5° in a powder X-ray diffraction spectrum.
3. The crystal according to Item 1 or 2, wherein the crystal is characterized by the powder X-ray diffraction spectrum shown in Fig. 1.
4. The crystal according to any one of Items 1 to 3, wherein the crystal has an endothermic peak at 170°C±5°C in differential scanning calorimetry analysis.
5. The crystal according to any one of Items 1 to 4, wherein the crystal is characterized by the differential scanning calorimetry (DSC) curve shown in Fig. 2 in differential scanning calorimetry analysis.

The present invention also provides a pharmaceutical composition comprising the crystal according to any one of the above items 1 to 5.

### Advantageous Effects of Invention

The present invention makes it possible to obtain a novel crystal of Compound (1) that can be obtained with sufficient reproducibility and that has excellent storage stability. The novel crystal of Compound (1) may be used as an active pharmaceutical ingredient for use in the manufacture of medicines.

### Brief Description of Drawings

Fig. 1 shows a powder X-ray diffraction spectrum of crystal I (the vertical axis represents intensity (counts). The horizontal axis represents diffraction angle (28±0.2°)).
Fig. 2 shows a differential scanning calorimetry (DSC) curve of crystal I. The horizontal axis represents temperature (°C). The vertical axis represents heat flow, endothermic, downward (mW). peak X = 169.89°C
Fig. 3 shows infrared absorption spectrum (IR) of crystal I.
Fig. 4 shows a fingerprint region of an infrared absorption spectrum (IR) of crystal I.
Fig. 5 shows a powder X-ray diffraction spectrum of crystal II (the vertical axis represents intensity (counts). The horizontal axis represents diffraction angle (2θ±0.2°)).
Fig. 6 shows a differential scanning calorimetry (DSC) curve of crystal II. The horizontal axis represents temperature (°C). The vertical axis represents heat flow, endothermic, downward (mW). peak X = 189.74°C
Fig. 7 shows a powder X-ray diffraction spectrum (initial value) of crystal I before a solid stability test (under exposure to light) (the vertical axis represents intensity (counts), and the horizontal axis represents diffraction angle (2θ±0.2°)).
Fig. 8 shows a powder X-ray diffraction spectrum (a value after irradiation at 3500000 lux/h) of crystal I after a solid stability test (under exposure to light) (the vertical axis represents intensity (counts), and the horizontal axis represents diffraction angle (2θ±0.2°)).
Fig. 9 shows a powder X-ray diffraction spectrum (initial value) of crystal II before a solid stability test (under exposure to light) (the vertical axis represents intensity (counts), and the horizontal axis represents diffraction angle (2θ±0.2°)).
Fig. 10 shows a powder X-ray diffraction spectrum (a value after irradiation at 3500000 lux/h) of crystal II after a solid stability test (under exposure to light) (the vertical axis represents intensity (counts), and the horizontal axis represents diffraction angle (2θ±0.2°)).
Fig. 11 shows a dynamic vapor sorption (DVS) system analysis (moisture adsorption-desorption test) of crystal I.
Fig. 12 shows a dynamic vapor sorption (DVS) system analysis (moisture adsorption-desorption test) of crystal II.

### Description of Embodiments

The present invention is more specifically explained below.

The present invention relates to a crystal of Compound (1). There are two kinds of crystal of Compound (1) of the present invention: crystal I and crystal II. Although both have excellent oral absorbability, crystal I is more preferable in terms of reproducibility in obtaining crystals, storage stability, and hygroscopic property.

In this specification, the term "crystal" is interpreted according to its general definition and designates a solid having a spatially regular arrangement of its atoms. Such a nature of crystal may be confirmed according to an X-ray diffraction spectrum.

In a powder X-ray diffraction pattern, the diffraction angle and the entire pattern are important in determining the identity of crystals due to the nature of data. Since the relative intensity of a powder X-ray diffraction pattern varies to some extent depending on the direction of crystal growth, the size of the particles, and measurement conditions, the relative intensity should not be strictly interpreted.

The values obtained from various patterns may contain error to some extent depending on the direction of crystal growth, the size of the particles, measurement conditions, and the like. Therefore, in this specification, the values of diffraction angle (2θ±0.2°) in a powder X-ray diffraction pattern may contain measurement error in a range of about ±0.2°.

The crystal of Compound (1) of the present invention may be produced through crystallization of amorphous state of Compound (1), or crystallization or recrystallization of a reaction product obtained after the synthesis of Compound (1).

Examples of Compound (1) to be used for the crystallization method of the present invention include a compound produced by the method disclosed in Patent Document 1. The crystallization may be performed using a liquid containing Compound (1) that results from the synthesis of Compound (1) or crystals (crude crystals) temporarily taken out from the liquid; however, to further improve the purity of the crystals, crystallization is preferably performed using crystals temporarily taken out from the liquid. The crystals to be temporarily removed may be crystal I or crystal II.

Examples of solvents to be used for the crystallization of crystal I of monohydrate of Compound (1) include, as a single solvent, ethyl acetate, n-propyl acetate, butyl acetate, or like ester-based solvents; acetone, methyl ethyl ketone, methyl isopropyl ketone or like ketone-based solvents; methanol, ethanol, or like alcohol-based solvents; acetonitrile; and the like. As mixed solvents, mixed solvents of any of the above solvents and water may be used. The solvent is more preferably an alcohol-based solvent, water, and a mixed solvent thereof, further preferably ethanol, water, and a mixed solvent thereof, and particularly preferably a mixed solvent of ethanol and water. The amount of solvent (v/w) is preferably not less than 5 times and not more than 30 times the amount of Compound (1), more preferably not less than 5 times and not more than 20 times the amount of Compound (1), further preferably not less than 7 times and not more than 15 times the amount of Compound (1). The dissolution temperature and the crystallization temperature are preferably not less than room temperature and not more than 100°C.

Further, the present invention may otherwise be expressed as crystal I produced by a method characterized by using a solvent selected from ester-based solvents, ketone-based solvents, alcohol-based solvents, acetonitrile, water, and mixed solvents thereof in crystallization. Furthermore, the present invention may also be expressed as a method for producing crystal I using a solvent selected from ester-based solvents, ketone-based solvents, alcohol-based solvents, acetonitrile, water, and mixed solvents thereof in crystallization.

To accelerate the crystallization of crystal I of monohydrate of Compound (1), an appropriate amount of crystal I of monohydrate of Compound (1) or a mixed crystal containing crystal I can be added as seed crystal. The amount of the seed crystal to be added is preferably 0.01 to 5 (w/v)%, and more preferably 0.03 to 1 (w/v)% based on the solvent amount. The crystal containing crystal I herein means a crystal containing crystal I in an amount of 25% or more. Further, crystallization may be performed under stirring so as to shorten the crystallization time and control the particle diameter.

The precipitated crystals may be isolated and purified from the solution in which the crystals are dissolved, the mixed solution, or the like, by a known isolation and purification means, such as filtration, washing with an organic solvent, or drying under reduced pressure. Examples of organic solvents to be used for washing include alcohol-based solvents, ketone-based solvents, and acetonitrile.

Crystal I is obtained either through crystallization of Compound (1) by using a single solvent such as an alcohol-based solvent, a ketone-based solvent, or the like, or crystallization by using a mixed solvent of the above solvent and water. Crystal II may be obtained by suspension washing using a single solvent such as a toluene/xylene-based solvent or an ester-based solvent having a high boiling point; crystal I may be obtained with significantly higher reproducibility than that of crystal II.

Crystal I of the present invention thus obtained has a powder X-ray diffraction spectrum shown in Fig. 1, and has a crystal structure. The characteristic diffraction angle (2θ±0.2°) of crystal I is at least two, preferably at least five, selected from 12.8°, 15.0°, 17.2°, 19.8°, 21.5°, 21.8°, 22.5°, 25.8°, and 27.5°. Crystal I further preferably has the powder X-ray diffraction spectrum shown in Fig. 1. Crystal I is a hydrate of Compound (I). In the present invention, a powder X-ray diffraction spectrum may be measured according to the test conditions shown in the Examples of the present invention.

Further, the term "near" used for the peak temperature of the endothermic peak in the differential scanning calorimetry (DSC) curve means substantially the same temperature, and preferably means a temperature in a range of ±5°C of the temperature. The term further preferably means a temperature in a range of ±2°C of the temperature. In the present invention, the differential scanning calorimetry (DSC) curve may be measured in accordance with the test conditions in the Examples in the present invention.

Further, Fig. 2 shows a differential scanning calorimetry (DSC) curve of crystal I. Crystal I has an endothermic peak near 170±5°C in a differential scanning calorimetry (DSC) curve, and preferably has the differential scanning calorimetry (DSC) curve shown in Fig. 2.

Crystal I thus obtained has, as a characteristic diffraction angle (2θ±0.2°), at least two peaks selected from 12.8°, 15.0°, 17.2°, 19.8°, 21.5°, 21.8°, 22.5°, 25.8°, and 27.5° in a powder X-ray diffraction spectrum, and also has an endothermic peak near 170±5°C in a differential scanning calorimetry (DSC) curve. More preferably, crystal I has, as a characteristic diffraction angle (2θ±0.2°), at least five peaks selected from 12.8°, 15.0°, 17.2°, 19.8°, 21.5°, 21.8°, 22.5°, 25.8°, and 27.5° in a powder X-ray diffraction spectrum, and also has an endothermic peak near 170±5°C in a differential scanning calorimetry (DSC) curve. Crystal I further preferably has a spectrum, such as the one shown in Fig. 1, in a powder X-ray diffraction spectrum, and also has a differential scanning calorimetry (DSC) curve, such as the one shown in Fig. 2.

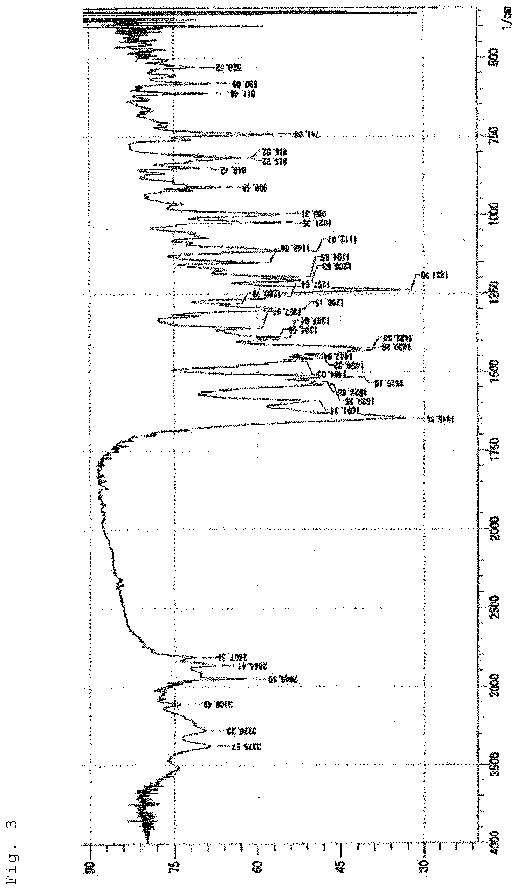

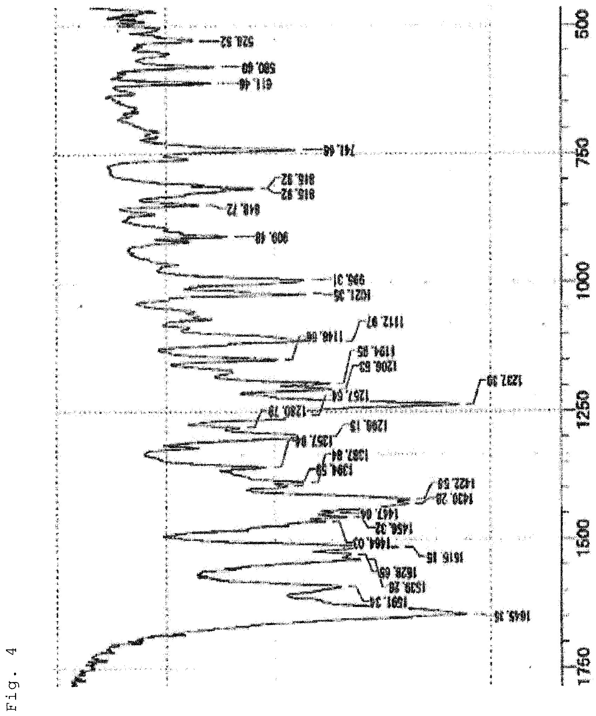

Crystal I thus obtained has, as a characteristic absorption band, 1645 cm⁻¹, 1237 cm⁻¹, 1021 cm⁻¹, 935 cm⁻¹, and 742 cm⁻¹ in an IR spectrum, and preferably has the IR spectrum shown in Fig. 3, and also has a fingerprint region having the IR spectrum shown in Fig. 4.

Further, in crystal I thus obtained, the chemical purity of Compound (1) is 99% or more, which may be measured by high-performance liquid chromatography (HPLC).

Crystal I of Compound (1) may be any crystal insofar as it contains crystal I. Crystal I may be a single crystal of crystal I or a polymorphic mixture containing other crystals. Crystal I preferably contains crystal I in an amount of 90 wt% or more, more preferably 95% or more.

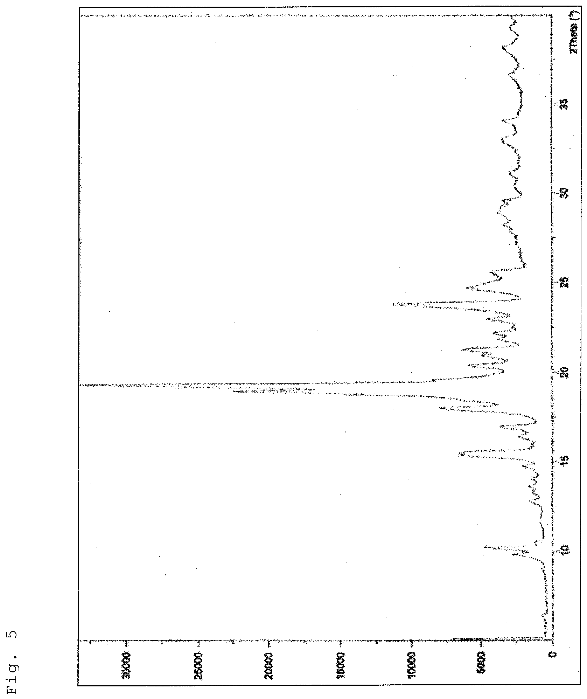

Crystal II has a powder X-ray diffraction spectrum, such as the one shown in Fig. 5. Crystal II has, as a characteristic diffraction angle (2θ±0.2°), two or more, preferably four or more, more preferably six or more, further preferably nine or more, further preferably ten peaks, selected from 5.1°, 10.2°, 15.3°, 15.6°, 18.0°, 19.0°, 19.3°, 20.4°, 23.8°, and 24.7°. Crystal II is a nonhydrate of Compound (I).

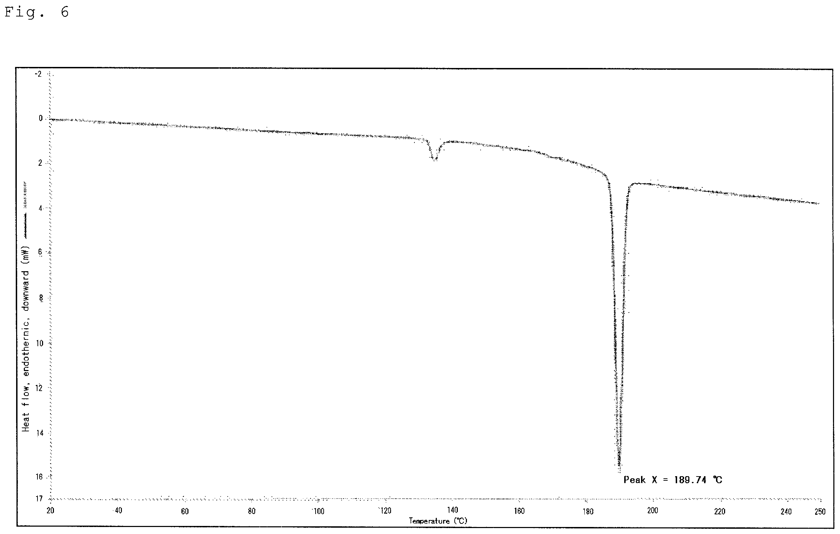

Further, Fig. 6 shows a differential scanning calorimetry (DSC) curve of crystal II. Fig. 6 reveals that crystal II has an endothermic peak near 190°C±5°C.

Further, for both crystal I and crystal II, the chemical purity was not decreased even after long-term storage under exposure to light. A powder X-ray crystal analysis revealed that, although the crystal form of crystal II changed after the storage, the crystal form of crystal I did not change, thereby showing its high storage stability. This showed that crystal I has particularly high storage stability.

Further, crystal I is less susceptible to the influence of humidity compared with crystal II, and thereby does not have significant restriction in storage conditions; however, crystal II is more susceptible to the influence of humidity and absorbs moisture, and thereby changes its weight. Due to such influence of moisture absorption, there is concern regarding maintaining the quality of crystal II.

Further, examples of solvents usable in the crystallization of crystal II include butyl acetate and like ester-based solvents, methyl isopropyl ketone and like ketone-based solvents, toluene, xylene, and like aromatic hydrocarbons; these solvents may be used as a single solvent or a mixed solvent. The crystallization temperature of crystal II is preferably not less than 100°C and not more than the boiling point of the solvent.

As is clear from the above, both crystal I and crystal II have high oral absorbability and long-term storage stability, and thus are useful as a raw material of an oral pharmaceutical composition. In particular, crystal I is superior in terms of long-term storage stability and hygroscopic property, as well as in reproducibility in obtaining crystals.

Crystal I or crystal II of the present invention may be processed, after being pulverized or without being pulverized, into various forms of pharmaceutical composition, for example tablets, capsules, granules, fine granules, powdered drug, dry syrup and like oral preparations, suppositories, inhalation agents, nasal drops, ointments, patches, aerosols and like external preparations, and injections; of these, oral preparations are preferable. These pharmaceutical compositions may be produced using a pharmaceutically acceptable carrier by a conventional preparation method known to a person skilled in the art. Oral solid preparations can be prepared as follows. An excipient, optionally together with a binder, disintegrant, lubricant, colorant, taste-masking or flavoring agent, etc., are added to the active ingredient to produce tablets, coated tablets, granules, powdered drugs, dry syrup, capsules, or the like, by using an ordinary method. Oral liquid preparations are produced as follows. A taste-masking agent, buffer, stabilizer, flavoring agent, etc. are added to the active ingredient to produce an internal liquid medicine, syrup, or the like by using an ordinary method. Injections are prepared as follows. A pH adjuster, buffer, stabilizer, isotonizing agent, topical anesthetic, etc. are added to the active ingredient to produce a subcutaneous injection, an intramuscular injection, or an intravenous injection by using an ordinary method. Suppositories are prepared as follows. An excipient, and, as necessary, a surfactant etc. are added to the active ingredient to produce a suppository by using an ordinary method. Ointments are prepared as follows. To prepare a paste, cream, or gel, an ordinary base, stabilizer, wetting agent, preservative, and the like, are added as required, and mixed and formulated into drugs by using an ordinary method. Examples of bases include white petrolatum, paraffin, glycerin, cellulose derivatives, polyethylene glycol, silicone and bentonite. Examples of preservatives include methyl parahydroxybenzoate, ethyl parahydroxybenzoate, and propyl parahydroxybenzoate. Patches can be prepared by coating a general support with the above ointment, cream, gel, paste, or the like by using an ordinary method. Suitable examples of supports include woven or nonwoven fabrics made from cotton, staple fibers, and chemical fibers; and films and foam sheets of soft polyvinyl chloride, polyethylene, polyurethane, and the like.

These pharmaceutical compositions are useful for allergic diseases, inflammatory diseases, myodegeneration diseases, and the like (Patent Document 1).

The amount of crystal I or II to be incorporated in the pharmaceutical composition may vary depending on the conditions of the patient to whom the pharmaceutical composition is administered, the dosage form thereof, and the like. However, in general, in the case of an oral agent, the amount of crystal I or II is about 0.05 to 1,000 mg per dosage unit form; in the case of an injection, the amount of crystal I or II is about 0.01 to 500 mg per dosage unit form; and in the case of a suppository or external preparation, the amount of crystal I or II is about 1 to 1,000 mg per dosage unit form. Further, the daily dose of crystal I or II in the pharmaceutical composition depends on the conditions of the patient, route of administration, age of the patient, and the like, and cannot be generalized. The daily dose is determined according to the doctor's prescription, and is generally about 0.05 to 5,000 mg.

### Examples

The present invention is more specifically explained below with reference to Examples; however, the present invention is not limited to these Examples. The present invention is fully described below by way of examples; however, it is understood that various changes and modifications by a skilled artisan are possible. Therefore, such changes and modifications are included in the present invention as long as they do not depart from the scope of the invention.

The various reagents used in the Examples were obtained from commercial suppliers, unless otherwise specified. NMR spectrum was obtained using AVANCEIII (400 MHz; Bruker BioSpin). The measurement was performed using tetramethylsilane as the internal reference when tetramethylsilane was contained in the deuterated solvent. In other cases, a NMR solvent was used as the internal reference. All δ values were expressed in ppm.

Each symbol stands for the following.

s: singlet
d: doublet
t: triplet
q: quartet
dd: double doublet
dt: double triplet
td: triple doublet
tt: triple triplet
ddd: double double doublet
ddt: double double triplet
dtd: double triple doublet
tdd: triple double doublet
m: multiplet
br: broad
brs: broad singlet

### Powder X-ray diffraction measurement

Powder X-ray diffraction was performed by lightly pulverizing an appropriate amount of a test substance as necessary using an agate mortar, and measuring the test substance in accordance with the following test conditions.

Device: PANalytical X'Pert PRO
Target: Cu
X-ray tube current: 30 mA
X-ray tube voltage: 40 kV
Scanning range: 2θ = 5.0 to 40.0°
Step: 2θ = 0.0170
Average time/step: 10.984 s
Variable divergence slit: irradiation length = 15 mm

Handling of the device, including data processing, was performed in accordance with the method and procedures specified for each device.

The values obtained from various spectra may vary to some extent depending on the direction of crystal growth, the size of the particles, measurement conditions, and the like. Therefore, the values should not be strictly interpreted.

### Thermal Analysis Measurement (differential scanning calorimetry (DSC) measurement)

DSC measurement was performed in accordance with the following test conditions.

Device: DSC8000, PerkinElmer Co., Ltd.
Sample: about 1.5 mg
Sample container: aluminum container
Temperature increase rate: increased to 250°C at 10°C/min
Atmospheric gas: nitrogen

Handling of the device, including data processing, was performed in accordance with the method and procedures specified for each device.

### Dynamic vapor sorption (DVS) system analysis

Dynamic vapor sorption (DVS) system analysis was performed under the following conditions.

Device: VTI SGA-100, TA Instruments
Sample: about 10 mg
Measurement temperature: 25°C
RH steps: 5-95-1% RH by 5% RH

### Elemental analysis

Elemental analysis was performed under the following conditions.

Device: Thermo Electron Flash EA 1112 series

Handling of the device, including data processing, was performed in accordance with the method and procedures specified for each device.

### Infrared absorption spectrum (IR)

Measurement of infrared absorption spectrum was performed under the following conditions.

Device: IR Prestige-21, SHIMADZU
Measurement method: KBr method

Handling of the device, including data processing, was performed in accordance with the method and procedures specified for each device.

### Example 1

### Synthesis of monohydrate of 4-((1-methylpyrrol-2-yl)-carbonyl)-N-(4-(4-morpholin-1-yl-carbonylpiperidin-1-yl)-phenyl)-1-piperazinecarboxamide (crystal I)

Ethanol (2680 g) and water (2100 g) were added to a crude product (425 g) of Compound (1) synthesized through the known method disclosed in Patent Document 1, and the mixture was heated to internal temperature of 70°C or more to be dissolved. Thereafter, cooling crystallization and stirring under ice cooling were performed, and then the resulting crystals collected by filtration were dried by heating under reduced pressure, thereby obtaining crystal I of Compound (1) as white powder. Mass of the obtained crystal: 342 g, Yield: 80.5%.

As shown in the powder X-ray diffraction spectrum in Fig. 1, 2θ of major peaks were 12.8°, 15.0°, 17.2°, 19.8°, 21.5°, 21.8°, 22.5°, 25.8°, and 27.5°. Further, as shown in the differential scanning calorimetry (DSC) curve in Fig. 2, the endothermic peak was 169.9°C.

1H-NMR (CDCl3): δ ppm 1.72-2.12 (4H, m), 2.47-2.78 (3H, m), 3.43-3.90 (18H, m), 3.80 (3H, s), 6.07-6.16 (1H, m), 6.33-6.39 (1H, m), 6.44 (1H, brs), 6.69-6.78 (1H, m), 6.86-7.02 (2H, m), 7.19-7.31 (2H, m)

Further, the elemental analysis resulted as follows and supports a monohydrate of 4-((1-methylpyrrol-2-yl)-carbonyl)-N-(4-(4-morpholin-1-yl-carbonylpiperidin-1-yl)-phenyl)-1-piperazinecarboxamide.
Calculation value: C27H38N6O5: C, 61.58; H, 7.27; N, 15.96.,
Actual measurement value: C, 61.57; H, 7.30; N, 15.86.

Further, Fig. 3 shows an infrared absorption spectrum, and Fig. 4 shows fingerprint region thereof. It was revealed that characteristic absorption bands are present at 1645 cm⁻¹, 1237 cm⁻¹, 1021 cm⁻¹, 935 cm⁻¹, and 742 cm⁻¹.

### Example 2

### Synthesis of crystal II of 4-((1-methylpyrrol-2-yl)-carbonyl)-N-(4-(4-morpholin-1-yl-carbonylpiperidin-1-yl)-phenyl)-1-piperazinecarboxamide

Butyl acetate (100 ml) was added to crystal I of Compound (1) (10.0 g). Thereafter, the mixture was heated under reflux for four hours using an oil bath set to 135°C, and then left for cooling. The solution was filtered and the resulting crystals were dried by heating under reduced pressure, thereby obtaining crystal II of Compound (1) as white powder. Mass of the obtained crystal: 8.97g, Yield: 89.7%.

As shown in the powder X-ray diffraction spectrum in Fig. 5, 2θ of major peaks were 5.1°, 10.2°, 15.3°, 15.6°, 18.0°, 19.0°, 19.3°, 20.4°, 23.8°, and 24.7°. Further, as shown in the differential scanning calorimetry (DSC) curve in Fig. 6, the endothermic peak was 189.7°C.

1H-NMR (CDC13): δ ppm 1.72-2.12 4H, m), 2.47-2.78 (3H, m), 3.43-3.90 (18H, m), 3.80 (3H, s), 6.07-6.16 (1H, m), 6.33-6.39 (1H, m), 6.44 (1H, brs), 6.69-6.78 (1H, m), 6.86-7.02 (2H, m), 7.19-7.31 (2H, m)

Further, elemental analysis resulted as follows and supports 4-((1-methylpyrrol-2-yl)-carbonyl)-N-(4-(4-morpholin-1-yl-carbonylpiperidin-1-yl)-phenyl)-1-piperazinecarboxamide. Calculation value: C₂₇H₃₆N₆O₄: C, 63.76; H, 7.13; N, 16.52. Actual measurement value: C, 63.69; H, 7.18; N, 16.42.

### Test Example 1

### Solid Stability Test (40°C, under light shielding)

Using crystal I obtained in the Examples, the solid stability after storage at 40°C under light shielding was measured.

Storage conditions: crystal I was stored at 40°C after being placed in a light shielding bottle covered with a lid. Measurement points: after 1 month, after 3 months, after 6 months

The amount of analog in the sample solution was measured by HPLC analysis. Handling of the device, including data processing, was performed in accordance with the method and procedures specified for each device (device: 2130-2450, HITACHI).
Column: Mightysil RP-18 GP 150-4.6 (5 µm)
UV detection: 220 nm
Column temperature: 40°C
Column flow rate: 1.0 mL/min
Mobile phase: A: acetonitrile, B: 10 mM phos. buffer
Gradient: Table 1

**Table 1**

| Time(min) | A | B |
|---|---|---|
| 0 | 2 0 % | 8 0 % |
| 2 | 2 0 % | 8 0 % |
| 2 0 | 7 0 % | 3 0 % |
| 2 7 | 7 0 % | 3 0 % |
| 2 8 | 2 0 % | 8 0 % |
| 3 5 | 2 0 % | 8 0 % |

Table 2 shows measurement of results of chemical purity. The chemical purity of crystal I was not changed after storage at 40°C under light shielding.

**Table 2**

| | Chemical Purity(%) |
|---|---|
| Initial Value | 99.8 |
| After 1 month | 99.8 |
| After 3 months | 99.6 |
| After 6 months | 99.9 |

### Test Example 2

### Solid Stability Test (under exposure to light)

Using crystals I and II obtained in the Examples, the solid stability after storage under exposure to light was measured.

Storage conditions: crystal I was stored at room temperature under exposure to light after being placed in a transparent glass dish covered with a lid.
Measurement point: after irradiation at 3500000 lux ▪ h (irradiation at 2000 lux × 1750 h)
Storage container: transparent glass dish

The powder X-ray diffraction upon the storage stability test was measured after an appropriate amount of the test substance was lightly pulverized as necessary using an agate mortar under the following test conditions.

Device: MiniFlex II, Rigaku Corporation
Target: Cu
X-ray output power: 15 mA, 30 kV
Scanning range: 3.0 to 40.0°
Step size: 0.010°
Scanning speed: 2.00°C/min.
Divergence slit: 1.25°
Scatter slit: open
Receiving slit: open

Handling of the device, including data processing, was performed in accordance with the method and procedures specified for each device.

The values obtained from various spectra may vary to some extent depending on the direction of crystal growth, the size of the particles, measurement conditions, and the like. Therefore, the values should not be strictly interpreted.

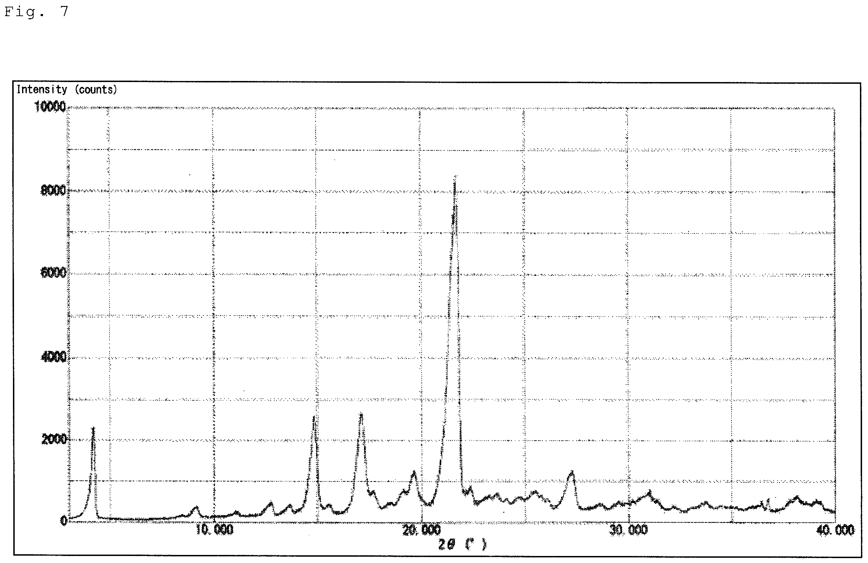

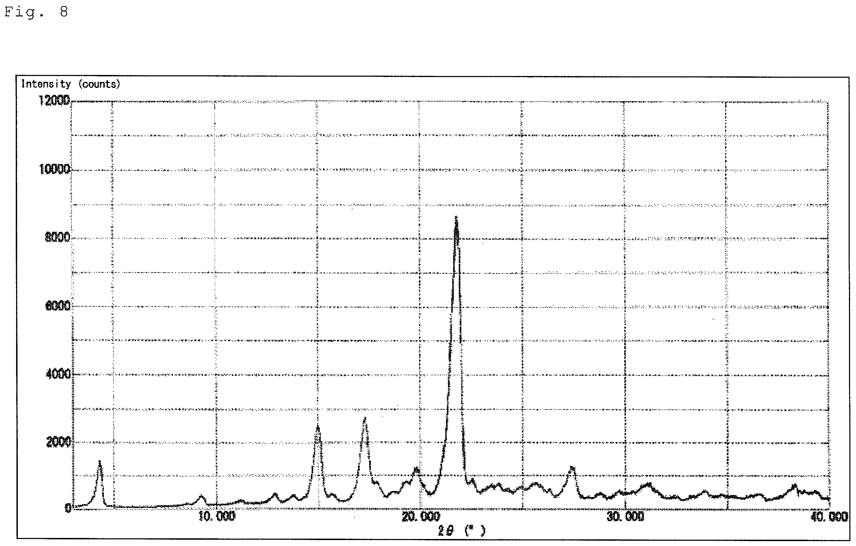

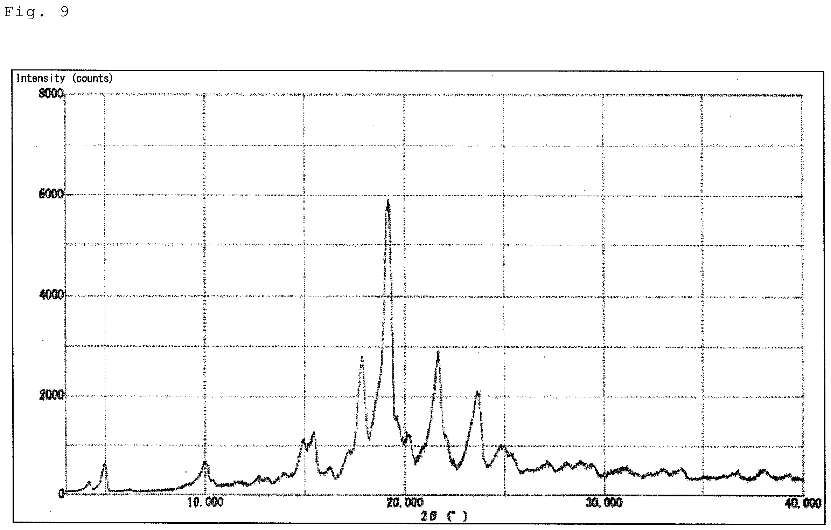

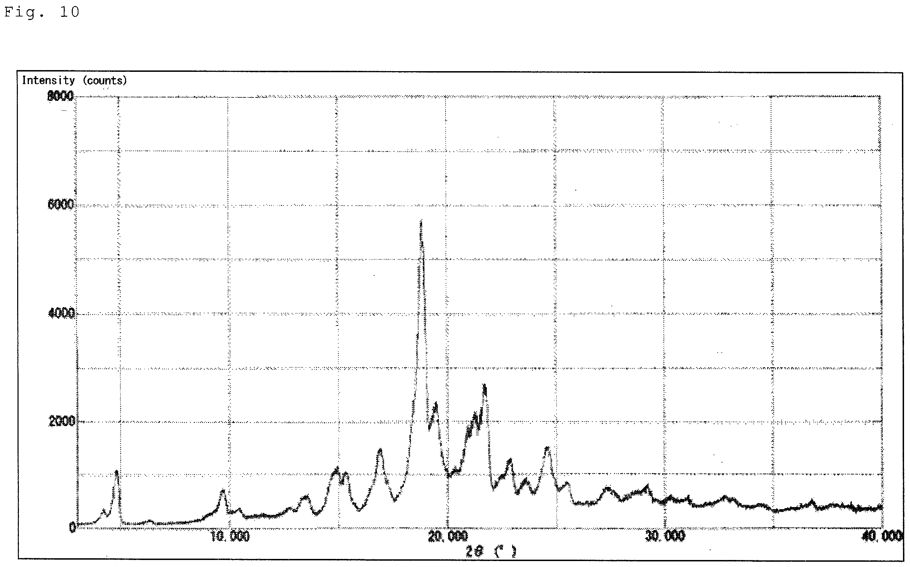

The results of powder X-ray crystal analysis of crystals I and II revealed that the crystal form of crystal I was not changed (Figs. 7 and 8), whereas the crystal form of crystal II was changed (Figs. 9 and 10). It was thus revealed that crystal I had excellent storage stability.

### Test Example 3

### Dynamic Vapor Sorption (DVS) System Analysis

Hygroscopic properties of crystals I and II obtained in the Examples were confirmed by dynamic vapor sorption (DVS) system analysis.

The adsorption and desorption data was collected at 5% RH intervals in a range of relative humidity (RH) of 5% to 95%. Before the measurement, the samples were heated under nitrogen purging to 25°C at 1°C/min, followed by drying for up to 6 hours until the weight change in 5 minutes became less than 0.0100%. The measurement was performed with equilibration for up to 2 hours until the weight change in 5 minutes became less than 0.0100%.

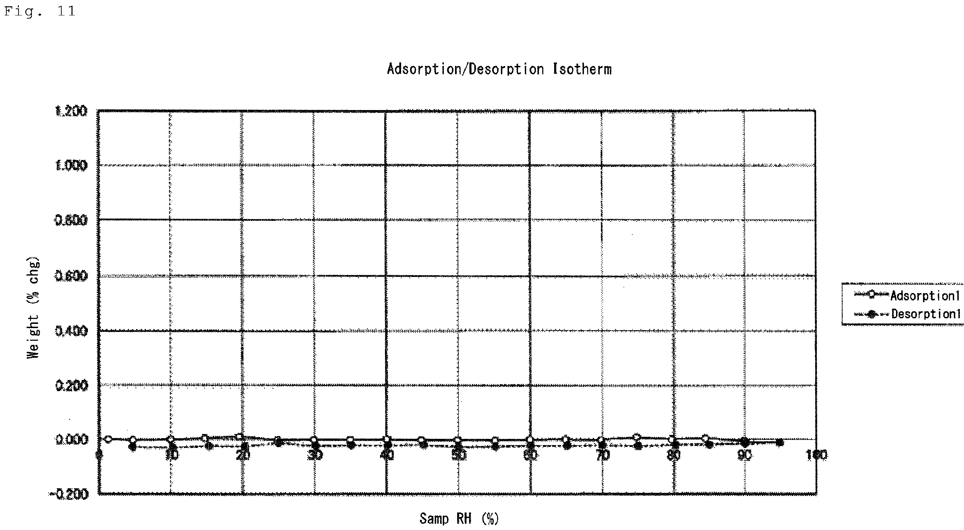

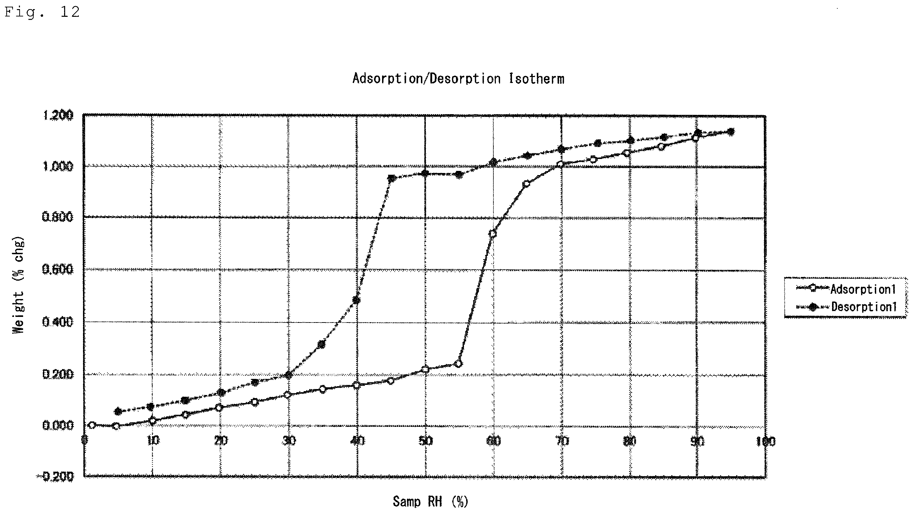

Figs. 11 and 12 show the results of dynamic vapor sorption (DVS) system analysis with regard to crystals I and II.

There were no changes in weight due to changes in humidity in crystal I; in contrast, a great change in weight due to a change in humidity was observed in crystal II. Further, for both crystals I and II, the results of powder X-ray crystal analysis were not changed before and after moisture adsorption and desorption.

It was thus revealed that crystal I was less susceptible to the influence of humidity, compared with crystal II.

## Claims

1. A crystal of monohydrate of 4-((1-methylpyrrol-2-yl)-carbonyl)-N-(4-(4-morpholin-1-yl-carbonylpiperidin-1-yl)-phenyl)-1-piperazinecarboxamide (Compound (1)) having at least 2 peaks of diffraction angle (28±0.2°) selected from 12.8°, 15.0°, 17.2°, 19.8°, 21.5°, 21.8°, 22.5°, 25.8°, and 27.5° in a powder X-ray diffraction spectrum, wherein the chemical purity of Compound (1) in the crystal is 99% or more, as measured by HPLC.

2. The crystal according to claim 1, wherein the crystal has at least 5 peaks of diffraction angle (29±0.2°) selected from 12.8°, 15.0°, 17.2°, 19.8°, 21.5°, 21.8°, 22.5°, 25.8°, and 27.5° in a powder X-ray diffraction spectrum.

3. The crystal according to claim 1 or 2, wherein the crystal is **characterized by** the powder X-ray diffraction spectrum shown in Fig. 1.

4. The crystal according to any one of claims 1 to 3, wherein the crystal has an endothermic peak at 170°C±5°C in differential scanning calorimetry analysis.

5. The crystal according to any one of claims 1 to 4, wherein the crystal is **characterized by** the differential scanning calorimetry (DSC) curve shown in Fig. 2 in differential scanning calorimetry analysis.

6. A pharmaceutical composition comprising the crystal according to any one of claims 1 to 5.

## Patentansprüche

1. Monohydrat-Kristall von 4-((1-Methylpyrrol-2-yl)-carbonyl)-N-(4-(4-Morpholin-1-yl-carbonylpiperidin-1-yl)-phenyl)-1-piperazincarboxamid (Verbindung (1)) mit mindestens 2 Beugungswinkel-Peaks (2θ ± 0.2°) in einem Pulverröntgenbeugungsspektrum, ausgewählt aus 12,8°, 15,0°, 17,2°, 19,8°, 21,5°, 21,8°, 22,5°, 25,8° und 27,5°, wobei die chemische Reinheit der Verbindung (1) im Kristall 99 % oder mehr beträgt, gemessen mittels HPLC.

2. Kristall gemäß Anspruch 1, wobei der Kristall in einem Pulverröntgenbeugungsspektrum mindestens 5 Beugungswinkel-Peaks (2θ ± 0,2°) aufweist, ausgewählt aus 12,8°, 15,0°, 17,2°, 19,8°, 21,5°, 21,8°, 22,5°, 25,8° und 27,5°.

3. Kristall gemäß Anspruch 1 oder 2, wobei der Kristall durch das in Fig. 1 gezeigte Pulverröntgenbeugungsspektrum gekennzeichnet ist.

4. Kristall gemäß einem der Ansprüche 1 bis 3, wobei der Kristall in der Differential-Scanning-Kalorimetrie-Analyse einen endothermen Peak bei 170°C ± 5°C aufweist.

5. Kristall gemäß einem der Ansprüche 1 bis 4, wobei der Kristall in der Differential-Scanning-Kalorimetrie-Analyse durch die in Fig. 2 gezeigte Differential-Scanning-Kalorimetrie (DSC)-Kurve gekennzeichnet ist.

6. Pharmazeutische Zusammensetzung, umfassend den Kristall gemäß einem der Ansprüche 1 bis 5.

## Revendications

1. Cristal de monohydrate de carboxamide 4-((1-méthylpyrrol-2yl)-carbonyl)-N-(4-(4-morpholin-1-yl-carbonylpipéridin-1-yl)-phényl)-1-pipérazine (Composé 1) présentant au moins 2 pics d'angle de diffraction (2θ ± 0,2°) sélectionnés parmi 12,8°, 15,0°, 17,2°, 19,8°, 21,5°, 21,8°, 22,5°, 25,8° et 27,5° dans un spectre de diffraction des rayons X sur poudre, dans lequel la pureté chimique du Composé (1) dans le cristal est de 99 % ou plus, telle que mesurée par CLHP.

2. Cristal selon la revendication 1, dans lequel le cristal présente au moins 5 pics d'angle de diffraction (2θ ± 0,2°) sélectionnés parmi 12,8°, 15,0°, 17,2°, 19,8°, 21,5°, 21,8°, 22,5°, 25,8° et 27,5° dans un spectre de diffraction des rayons X sur poudre.

3. Cristal selon la revendication 1 ou 2, dans lequel le cristal est **caractérisé par** le spectre de diffraction des rayons X sur poudre représenté sur la figure 1.

4. Cristal selon l'une quelconque des revendications 1 à 3, dans lequel le cristal présente un pic endothermique à 170 °C ± 5 °C en analyse calorimétrique différentielle.

5. Cristal selon l'une quelconque des revendications 1 à 4, dans lequel le cristal est **caractérisé par** la courbe d'analyse calorimétrique différentielle (ACD) représentée sur la figure 2 en analyse calorimétrique différentielle.

6. Composition pharmaceutique comprenant le cristal selon l'une quelconque des revendications 1 à 5.
